Europäisches Patentamt

European Patent Office

Office européen des brevets

(19)

(11) Publication number: 0 669 082 A1

# EUROPEAN PATENT APPLICATION

(12)

(21) Application number: 95200247.5

(22) Date of filing: 02.02.95

(51) Int. Cl.⁶: A21D 8/04, C12N 9/96

(30) Priority: 23.02.94 EP 94200446

(43) Date of publication of application:
30.08.95 Bulletin 95/35

(84) Designated Contracting States:
AT BE CH DE DK ES FR GB GR IE IT LI NL PT
SE

(71) Applicant: UNILEVER N.V.
P.O. Box 760
NL-3000 DK Rotterdam (NL)

(84) BE CH DE DK ES FR GR IT LI NL PT SE AT

(71) Applicant: UNILEVER PLC
Unilever House
Blackfriars
P.O. Box 68
London EC4P 4BO (GB)

(84) GB IE

(72) Inventor: Roza, Martinus
Olivier van Noortlaan 120
NL-3133 AT Vlaardingen (NL)
Inventor: Pieters, Guida Hubertina E.
Abel Tasmanlaan 30
NL-3133 AB Vlaardingen (NL)

(74) Representative: Hartong, Richard Leroy
Unilever N.V. Patent Division
P.O. Box 137
NL-3130 AC Vlaardingen (NL)

(54) Storage-stable enzyme solutions.

(57) Aqueous bread improver compositions with a long shelf life comprise :
  - an effective amount of a water-soluble food grade oxidant in an amount up to its saturation;
  - an effective amount of at least one water-soluble bread-improving enzyme;
  - 0-40 wt.% of a lecithin,
  - while the pH of the solution is 3.0-7.0.

EP 0 669 082 A1

Rank Xerox (UK) Business Services
(3.10/3.09/3.3.4)

It is known from the prior art (EP 421,510 and EP 572,051) that liquid bread improvers provide a considerable advantage over non-liquid bread improver systems. However, the liquid bread improvers in the above-mentioned citations are based on liquid fat components, which are not always allowed in such systems, and not in every country. Moreover, the presence of fats is nowadays not always considered a desired ingredient.

Therefore, we have studied how to make liquid bread improvers in which no fat component is present. The liquid character of such bread improvers therefore had to be contributed by the solvent. Although water seemed a very suitable solvent for such systems, it could be expected on the basis of prior art disclosures that the keepability (shelf life) of aqueous enzyme solutions would be short. For example, US 4,548,727 discloses (column 1, lines 36-42) that enzymes are inherently unstable in the presence of water, resulting in a rapid decrease in enzymatic activity. In order to increase the shelf life of an aqueous enzyme solution, esters having the formula $RCOOR^1$ (R and $R^1$ being both alkyl) are added to the enzyme solution. Enzymes stabilised in this way are : proteases, amylases and mixtures thereof. However, adding such an ester involves an increase in the costs, it is not always allowed as food ingredient in bread improver systems and it means addition of an ingredient that has no functionality in the dough, while the process is complicated because of the addition of an extra ingredient not akin to the dough system.

In US 4,243,546 and US 4,532,064 other routes for the stabilisation of aqueous enzyme compositions for the detergent area are given. According to these prior art documents, an alkanol amine is added in combination with an (organic) acid, or an organic dicarboxylic acid is added. The additives used, however, cannot be applied in food products, such as bread doughs, and/or have no functionality in the doughs.

According to US 5,039,430 a fresh aqueous solution of an acid-stable enzyme is made, whereupon the solution is added to the dough (column 6, lines 24-41). A similar teaching can be found in US 5,209,938. Nothing is disclosed about an aqueous enzyme solution with a good shelf life (i.e. about 6 months at ambient temperature) wherein no additives, such as organic alkyl esters, dicarboxylic acids or alkanol amines, are present.

We have now found novel aqueous enzyme solutions with a good shelf life wherein an additional bread-improving component is present, such as a derivative of ascorbic acid or $KBrO_3$, so that the solutions can be used as a bread improver system. These novel enzyme solutions are free of additives, not allowed in and/or not akin to dough systems.

The long shelf life is determined by either adding a preservative, such as K-sorbate or benzoate or acetate salts, or by subjecting the enzyme solution to a sterilisation treatment, avoiding, however, high temperatures (as enzymes cannot stand heat treatment).

As the use of preservatives, such as K-sorbate, is not always allowed in food compositions, sterilisation is a useful alternative to achieve long shelf lives.

Accordingly, our invention concerns an aqueous bread improver composition having a long shelf life, preferably sterilised, wherein the composition comprises:

- an effective amount of a water-soluble, food grade oxidant, such as a derivative of ascorbic acid or an alkali bromate salt, in an amount up to its saturation;
- an effective amount of at least one water-soluble bread-improving enzyme;
- 0 - 40 wt.% of a lecithin, optionally modified, or a lecithin fraction,

while the pH of the aqueous composition ranges from 3.0 to 7.0. These compositions are free of additives not allowed in and/or not akin to dough systems.

It was found, that the presence of the oxidant, in particular of ascorbic acid had an advantageous impact on the shelf life. The higher the concentration of ascorbic acid in our compositions the longer was the shelf life.

It was further found that the best products with the longest shelf lifes were obtained, if the compositions were produced and stored in such a way that contact with oxygen was avoided to the greatest extend.

The bread-improving enzyme can be selected from the group consisting of amylase, acid-resistant amylase, trans-glutaminase, hemicellulase, oxidases, peroxidases, protease and lipase.

Preferred enzymes are α-amylase, acid-stable α-amylase and xylanase (a hemicellulase).

Preferred compositions comprise bread improver compositions wherein at least one of the following enzymes are present in the amounts indicated:

(-) 0 - $10^8$, preferably $5 \times 10^3$ - $10^5$ units of amylase per liter composition;
(-) 0 - $10^{10}$, preferably $10^5$ - $10^8$ units of hemicellulase, in particular of xylanase per liter composition;
(-) an effective amount of oxidase, glucose peroxidase, lipoxygenase, protease, transglutaminase or lipase.

The amount of the ascorbic acid derivative is effectively 0.1-20 wt.%, while the ascorbic acid derivative is selected from at least one component of the group consisting of ascorbic acid, alkali salts of ascorbic acid, ammonium salts of ascorbic acid and alkali earth metal salts of ascorbic acid.

The pH of the aqueous solution has a profound impact on its stability. Compositions with pH ≤ 4 had only to be pasteurised while compositions with pH of 3-4 could be sterilised and those with pH ≥ 4 had to be sterilised in order to achieve the required shelf life.

The above-mentioned pH requirement also had another consequence because the different bread-improving enzymes have a different sensitivity to pH. Therefore, aqueous compositions with pH of 3-6 can contain enzymes selected from the group of acid-resistant amylase, xylanase and oxidase. Aqueous compositions with pH of 6.0-7.0 can contain amylase, xylanase and oxidase.

As disclosed above, the aqueous enzyme solution can contain 0-40 wt.% of a lecithin. Depending on its application, the presence of the lecithin is sometimes advantageous to the properties of the dough to which it is added, in particular dough strength and shelf life of the baked products are improved.

Because of the fact that a long shelf life for the aqueous composition is normally obtained at pH > 4 by sterilisation treatment, and lecithin in water can only be sterilised by heat treatment, in which case the lecithin must be a defatted lecithin (a fat-containing lecithin displays flocculation upon heating in water), we prefer our aqueous enzyme solution to contain a defatted lecithin containing less than 5 wt.% of total monoglycerides, diglycerides and triglycerides (calculated on lecithin).

The most preferred compositions according to our invention comprise :

(-) 95 - 99.5 wt.% of water;

(-) 0.5 - 5 wt.% of ascorbic acid or its alkali salt;

(-) an effective amount of a bread-improving enzyme,

(-) its pH being 3 - 6.

It should be understood that in the context of the disclosure above, the following definitions are used :

1 unit of $\alpha$-amylase is the amount of enzyme producing that amount of reducing groups equivalent to 1 micromol of glucose per minute at 40°C and pH of 5.0, using a 1% soluble starch solution as substrate.

1 unit of hemicellulase is the amount of enzyme producing that amount of reducing groups equivalent to 1 microgram of xylose per minute at 40°C and pH of 5.0, using a 1% soluble oatspelt xylan solution as substrate.

A long shelf life and acceptable stability for component X present in the aqueous bread improver solution for a given temperature and acidity can be described, using the following relation :

$$[\text{Component X}]_{(t=0,T,pH)} = [\text{Component X}]_{(t,T,pH)} * K * t$$

where

$$K = \text{degradation rate of component X over time}$$
$$t = \text{required shelf life}$$

$$[\text{Component X}]_{(t,T,pH)} = \text{residual component concentration after time t,}$$
$$[\text{Component X}]_{(t=0,T,pH)} = \text{initial component concentration at } t = 0,$$

Component X being a bread-improving enzyme, ascorbic acid or its derivative(s).

Critical for the suitability of each active component X in such system is that both initial and residual concentration guarantee good-quality baked products, without changing the bread improver application level or application procedure.

The bread improver is considered sufficiently stable if the above holds for a time period of 6 months at 20°C or 4 months at 30°C.

The preservative, such as K-sorbate can be added in amounts up to 1.5 wt.%, either to the unsterilised enzyme solution or to the sterilised enzyme solution.

The shelf life of the enzyme solutions can easily be at least 6 months at 25°C.

Depending on the bread improver application, other water-soluble ingredients that do not adversely affect the shelf life of the other vital components can be added; examples of these other components are vitamins and malt extracts.

As stated previously, enzymes are not resistant to heat; therefore, the enzyme solution cannot be sterilised by heat treatment.

It was found that sterilisation by microfiltration was very effective and could ensure the required shelf life.

Such a low-temperature sterilisation also has the advantage that the ascorbic acid derivative can be present in the enzyme solution to be sterilised. This is beneficial because the oxidative degradation of ascorbic acid and its derivatives is known to be enhanced by higher temperatures (cf. Analytical Chem. 57, 1985, pp. 555-558; Analyst (1989), 114, pp. 169-171; J. Agric. Food Chem. 31 (1983), 980-985).

The low-temperature sterilisation will result in enzyme solutions with a long shelf life, wherein high levels of ascorbic acid derivatives can be present.

Therefore, in one aspect of the invention our sterilised aqueous bread improver compositions are made by mixing of water, ascorbic acid derivative and bread-improving enzymes, adjusting the pH either prior to or after the addition of the enzymes to 3.0 - 7.0, if necessary, filtering and sterilising the mixture by passing it through a micro filter having a pore size of less than 0.22 $\mu$m, and packing the mixture aseptically.

However, in the above-mentioned process, lecithin cannot be present because the microfiltration cannot be performed in the presence of lecithin. For the preparation of a lecithin-containing product the following process had to be carried out :

(1) water, ascorbic acid derivative and bread-improving enzyme are mixed;

(2) the pH is adjusted to 3.0 - 7.0, if necessary;

(3) the mixture is sterilised by performing microfiltration, using a filter with a pore size of less than 0.22 $\mu$m;

(4) water and defatted lecithin are mixed;

(5) the mixture, resulting from step (4) is pasteurised or sterilised;

(6) a cooled, pasteurised or sterilised mix obtained after step (5) is mixed with the sterilised mixture obtained after step (3);

(7) the resulting mixture of step (6), optionally after filtration, is packed aseptically.

It should be understood, that a pasteurization can be performed as a heat treatment, i.e. 2-30 min. at 55-80°C. Sterilization can be performed as a heat treatment as well, however applying temperatures of 135-165°C during 1 sec. to 2 min.

Doughs containing the bread improver composition according to the invention are also part of the invention.

Our compositions can be packed in flexible package material. In order to avoid contact between our compositions and oxygen the package material preferably is impermeable for oxygen.

Another use of the aqueous bread improver compositions is to apply them as a water phase for the production of bakery emulsions, in particular water-continuous systems, as these are also known to suffer from microbiological degradation. The fat content of such products can range from 10-85 wt.%.

EXAMPLES

1. Preparation of liquid bread improver

1.1 Liquid bread improver, free of lecithin, not sterilised

Ascorbic acid (1.0%) and potassium sorbate (0.13%) are added to water at ambient temperature, with intensive mixing. Aqueous concentrated solutions of acid-stable alpha amylase and xylanase are added to the solution in such a way that the resulting final concentrations of the amylase and xylanase are equal to 50,000 units, respectively 7.5 $10^6$ units per litre of bread improver. The bread improver is stored at ambient temperature.

1.2 Liquid bread improver, free of lecithin, sterilised

Ascorbic acid (1.0%) is added to water at ambient temperature, with intensive mixing. Aqueous solutions of acid-stable alpha amylase and xylanase are added to the solution in such a way that the resulting final concentrations of the amylase and xylanase are equal to 50,000 units, respectively 7.5 $10^6$ units per litre of bread improver. The solution is sterilised by filtering it through a 0.22 micron filter. The bread improver is stored at ambient temperature in sterile containers.

1.3 Liquid bread improver, with lecithin, sterilised

Defatted lecithin (11%) is emulsified in water, using high shear at ambient temperature. The emulsion is sterilised at 120°C for 15 minutes to ensure microbial stability of this emulsion. After sterilisation, the emulsion is cooled again to ambient temperature. All following handling procedures involving the bread improver must be performed under aseptic conditions to avoid contamination and microbial risks.

A highly concentrated ascorbic acid solution (20%) is sterilised by filtering the solution, using a 0.22 micron filter. This solution is added to the lecithin emulsion in such a way that the final lecithin emulsion contains 1% of ascorbic acid. The emulsion is neutralised, using microfiltered (to ensure sterility) 0.5 N sodium hydroxide to a pH equal to 6.5. In doing so, the ascorbic acid is neutralised to its sodium salt. The neutral pH enables the use of α-amylase instead of acid-stable α-amylase.

A concentrated enzyme solution containing α-amylase (1 million units per liter) and xylanase (150 million units per litre) is sterilised, using a microfilter of 0.22 microns. This enzyme solution is added to the lecithin emulsion in such a way that the enzyme activity of the final lecithin emulsion is 50,000 units α-amylase and 7.5 million units xylanase per liter of bread improver.

The final lecithin emulsion contains 9.9% of lecithin, 1% sodium ascorbate, 50,000 α-amylase units per liter and 7.5 million xylanase units per liter and is stored at ambient temperature in sterile containers.

2. Preparation of french standard bread

2.1 Recipe

2.1.1 Ingredients

| Flour (Parel ex Meneba) Yeast (Konings) Salt | 1000 grams 26 grams 22 grams | Temperature : 22°C |
|---|---|---|
| Water Bread improver | 600 grams 10 grams | Temperature : 0°C |

2.1.2 Process

Room temperature 22°C

2.1.2.1 Mixing

Mixer : Eberhardt Minimat 10

Flour, water and bread improver are added to the kneader. The ingredients are kneaded at low speed (i.e. 9.5 rpm. for the vessel and 75 rpm. for the spiral) for 3 minutes. Immediately after the slow-speed kneading, the mixture is kneaded for 8 minutes at high speed (i.e. 19 rpm. for the vessel and 150 rpm. for the spiral). During these 8 minutes, yeast is added after 4 minutes and the salt is added after an additional 2 minutes. The complete mixture is mixed during the final 2 minutes.

2.1.2.2 Proofing

| Duration of proofing : | first second third | 20 minutes 20 minutes 120 minutes |
|---|---|---|

| Relative humidity of proofing cabinet Proofing temperature | 70% RH 26°C |
|---|---|

### 2.1.2.3 Baking

| steaming time 3 seconds at beginning of baking | |
|---|---|
| temperature | 240 ° C upper setting<br>225 ° C lower setting |
| baking time | 18 minutes |

### 2.3 Results

The liquid bread improver was intended for the french standard bread and the above-mentioned Examples 1.1. to 1.3 were evaluated, using a currently used powdered bread improver for the french standard bread, i.e. Softine ex FraBeP (France). The liquid bread improvers and softine were dosed at 1% with respect to the flour, evaluated fresh and after a storage time of 6 months at ambient temperature (20 ° C). The results are compiled in the following Table :

| B.I. sample | 1.1 fresh | 1.1 after storage | 1.2 fresh | 1.2 after storage | 1.3 fresh | 1.3 after storage | Softine (ref.) |
|---|---|---|---|---|---|---|---|
| **Evaluation of dough after mixing** | | | | | | | |
| Dough temperature (°C) | 26.0 | 25.8 | 26.0 | 26.0 | 25.7 | 25.9 | 26.0 |
| Stickiness (1 = sticky, 5 = dry) | 3 | 2.5 | 2.5 | 3 | 3 | 3 | 3 |
| Firmness (1 = slack, 5 = firm) | 2.5 | 3 | 2.5 | 3 | 3 | 2.5 | 3 |
| Extensibility (1 = relaxed, 5 = stiff) | 3 | 3 | 3 | 3 | 3 | 3 | 3 |
| Behaviour (1 = relaxed, 5 = stiff) | 3 | 3 | 2.5 | 3 | 3 | 2.5 | 3 |
| **Evaluation of dough after first and second proofing** | | | | | | | |
| Stickiness (1 = sticky, 5 = dry) | 2.5 | 2.5 | 3 | 3 | 3 | 2.5 | 3 |
| Firmness (1 = slack, 5 = firm) | 3 | 3 | 2.5 | 3 | 3 | 3 | 3 |
| Extensibility (1 = relaxed, 5 = stiff) | 2.5 | 3 | 3 | 3 | 3.5 | 3 | 3 |
| **Evaluation of unbaked fully proved dough** | | | | | | | |
| Fermentation stability | good | good | good | good | good | good | good |
| **Evaluation of baked product** | | | | | | | |
| Specific volume (ml/g) | 8.3 | 8.2 | 8.5 | 8.1 | 8.6 | 8.5 | 8.4 |
| Surface splitting (1 = no, 5 = much) | 4 | 3.5 | 3.5 | 3.5 | 4.5 | 4.5 | 4.5 |
| Crust crispiness (1 = crispy, 5 = tough) | 1.5 | 1.5 | 1 | 1 | 1.5 | 1 | 1 |
| Crust colour (1 = light, 5 = dark) | 4 | 4 | 3.5 | 4 | 4 | 4 | 4 |
| Crumb structure (1 = fine, 5 = coarse) | 2 | 2 | 2 | 2 | 2.5 | 2 | 2 |
| Overall structure (1 = regular, 5 = irregular) | 2 | 3 | 2 | 2.5 | 2.5 | 2 | 2.5 |
| Crumb softness (1 = soft, 5 = firm) | 1 | 1 | 1 | 1 | 1.5 | 1 | 1 |

## Claims

1. Aqueous bread improver composition having a long shelf life, preferably sterilised, wherein the composition comprises:

EP 0 669 082 A1

- an effective amount of a water-soluble, food grade oxidant, such as a derivative of ascorbic acid or an alkali bromate salt, in an amount up to its saturation;
- an effective amount of at least one water-soluble bread-improving enzyme;
- 0 - 40 wt.% of a lecithin, a modified lecithin or lecithin fraction;
  while the pH of the aqueous composition ranges from 3.0 to 7.0.

2. Aqueous bread improver composition according to Claim 1, wherein the bread-improving enzyme is selected from the group consisting of amylase, acid-resistant amylase, hemicellulase, oxidases, peroxidases, protease and lipase.

3. Aqueous bread improver composition according to Claim 1 or 2, wherein at least one of the following enzymes are present in the amounts per liter composition indicated:
   (-) 0 - $10^8$ units of amylase;
   (-) 0 - $10^{10}$ units of hemicellulase, in particular of xylanase;
   (-) an effective amount of oxidase, glucose peroxidase, lipoxygenase, protease or lipase.

4. Aqueous bread improver composition according to Claim 1, wherein the amount of the ascorbic acid derivative is 0.1 - 20 wt.%.

5. Aqueous bread improver composition according to Claim 1 or 4, wherein the ascorbic acid derivative is selected from at least one component of the group consisting of ascorbic acid, alkali salts of ascorbic acid, ammonium salts of ascorbic acid and alkali earth metal salts of ascorbic acid.

6. Aqueous bread improver composition according to Claims 1-5, wherein the composition is pasteurised if its pH $\leq$ 4, or sterilised if its pH > 3, preferably > 4.

7. Aqueous bread improver composition according to Claim 1, wherein the pH of the composition is 3 - 6.0, while the bread-improving enzyme is selected from at least one enzyme of the group consisting of acid-resistant amylase, xylanase, and an oxidase.

8. Aqueous bread improver composition according to Claim 1, wherein the pH of the composition is 6.0 - 7.0, while the bread-improving enzyme is selected from xylanase and an oxidase.

9. Aqueous bread improver composition according to Claims 1-8, wherein the lecithin is a defatted lecithin containing less than 5 wt.% of total monoglycerides, diglycerides and triglycerides (calculated on lecithin).

10. Aqueous bread improver composition comprising:
    (-) 95 - 99.5 wt.% of water;
    (-) 0.5 - 5 wt.% of ascorbic acid or its alkali salt;
    (-) an effective amount of a bread-improving enzyme,
    (-) its pH being 3 - 6.

11. Aqueous bread improver composition according to Claims 1-10, wherein the composition has a shelf life of at least 6 months at 25°C.

12. Aqueous composition according to Claim 1, wherein the composition also contains up to 1.5 wt.% of a preservative, in particular K-sorbate.

13. Doughs comprising an effective amount of the bread improver composition according to Claims 1-12.

14. Process for the preparation of a sterilised aqueous bread improver composition according to Claims 1-12, wherein water, ascorbic acid derivative and bread-improving enzymes are mixed, the pH of the mixture is adjusted to 3.0 - 7.0, if necessary, the mixture is filtered and sterilised by passing it through a microfilter with a pore size of less than 0.22 $\mu$m, whereupon the sterilised mixture is packed aseptically.

8

**15.** Process for the prepearation of a pasteurised aqueous bread improver composition according to Claims 1-12, wherein:

(1) water, ascorbic acid derivative and bread-improving enzyme are mixed;

(2) the pH is adjusted to 3.0 - 7.0, if necessary;

(3) the mixture is sterilised by performing microfiltration, using a filter with a pore size of less than 0.22 $\mu$m;

(4) water and defatted lecithin are mixed;

(5) the mixture, resulting from step (4) is pasteurised or sterilised;

(6) a cooled, pasteurised or sterilised mix obtained after step (5) is mixed with the sterilised mixture obtained after step (3);

(7) the resulting mixture of step (6), optionally after filtration, is packed aseptically.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP-A-0 109 244 (KYOWA HAKKO KOGYO CO., LTD.)<br>* page 3, line 12 - page 5, line 14; claims * | 1-5,7,9,10,13 | A21D8/04<br>C12N9/96 |
| Y | WO-A-89 08403 (CPC INTERNATIONAL INC.)<br><br>* page 9, paragraph 1 - last paragraph *<br>* page 10, paragraph 2 * | 1-5,7,9,10,13 | |
| D,A | EP-A-0 419 907 (ENZYME BIO-SYSTEMS LTD.)<br>* page 4, line 3 - line 23; example 4 * | 1,2 | |
| A | EP-A-0 468 731 (ORIENTAL YEAST CO., LTD.)<br><br>* page 2, line 47 - page 10, line 13 * | 1-5,7,8,10,13 | |
| A | EP-A-0 338 452 (CULTOR LTD.)<br><br>* page 3, line 41 - page 4, line 58 * | 1-5,7-10,13 | |

TECHNICAL FIELDS
SEARCHED      (Int.Cl.6)

A21D
C12N

The present search report has been drawn up for all claims

| Place of search | Date of completion of the search | Examiner |
|---|---|---|
| THE HAGUE | 3 May 1995 | Bevan, S |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)